# EUROPEAN PATENT APPLICATION

(11) **EP 2 012 126 A1**
(43) Date of publication of application: **07.01.2009**
(21) Application number: 07111694.1
(22) Date of filing: 04.07.2007
(51) Int. Cl.: G01N 33/543

(54) **Porous biological assay substrate and method for producing such substrate**

(71) Applicant: Koninklijke Philips Electronics N.V., 5621 BA Eindhoven (NL)
(72) Inventor: The designation of the inventor has not yet been filed
(74) Representative: Plünnecke, Ingo

(57) **Abstract**

The invention provides a porous biological assay substrate suitable for detecting at least one analyte in at least one sample fluid. The substrate is provided with capture probes in plural spot areas, the capture probes being able to each specifically bind at least one target analyte. The substrate comprises a top layer provided with a plurality of holes in a pattern that matches the spot areas. The substrate thereby shows improved binding efficiency. The invention also relates to a method and device for producing the biological assay substrate, and to a method for examining analyte fluids using the substrate.

## Description

### FIELD OF THE INVENTION

The present invention relates to the field of analysing biological sample fluids with respect to certain analytes and in particular to a porous substrate to be used for a biological assay suitable for detecting at least one analyte in a biological sample fluid. In particular, the present invention permits an accurate and efficient analysis of biological sample fluids. The invention further relates to a method for producing a biological assay substrate by depositing a plurality of substances onto the substrate, and to a biological assay substrate obtainable by the method. The present invention also relates to a method for analysing a sample fluid with respect to one or more analyte molecules present in the sample fluid. The analyte may comprise any compound capable of binding to capture probes on the substrate, including target biological compounds, proteins, DNA, and so on. The method can be used for molecular diagnostic tests, e.g. for measuring the presence of genes of infectious disease pathogens.

### BACKGROUND OF THE INVENTION

Arrays of capture probes on a substrate are used in biological test assays, for instance to examine analyte biological fluids, such as human blood or tissue samples, for the presence and/or concentration of certain bacteria, viruses and/or fungi. The capture probes have a selective binding capacity for a predetermined indicative factor, such as a protein, DNA or RNA sequence that belongs to a specific bacterium, virus or fungus. In the micro array technique, a set of specific capture probes, each of which being chosen in order to interact specifically (e.g. hybridize in the case of a DNA microarray) with one particular target biological compound, are deposited at specific locations of a biosensor solid substrate, for instance by printing, and immobilized. After the substrate has been provided with the capture probes, the analyte fluid is forced to flow through the substrate, or forced to flow over the substrate. In order to be able to visualize the presence of an indicative factor in the analyte fluid, molecules of the indicative factors in the analyte fluid may for instance be provided with fluorescent and/or magnetic labelling. In case of an ELISA (enzyme-linked immunosorbent assay) an enzyme is attached to the second antibody. An intensely colored or fluorescent compound is then formed by the catalytic action of this enzyme. The (labelled) molecules of the analyte fluid adhere to those capture probes of the substrate that have binding capacity for the molecule considered (e.g. hybridize in the case of a DNA microarray). This results in a detectable fluorescence on the spot the specific factor adheres to, at least when using fluorescent labelling. The captured molecules are typically read by illumination with a light source, and the fluorescent pattern recorded with the aid of a CCD camera for instance. The recorded pattern is a characteristic of the presence of a bacterium or a set of bacteria. By providing capture probes with different specificity for different factors, the array may be used to assay for various different factors at the same time. Using such arrays enables high-throughput screening of analyte fluids for a large amount of factors in a single run.

There is a continuing trend to increase the number of spots (up to 1000 or more) of an increasing amount of different bioactive materials (100 or more). Moreover, the lateral dimensions of the substrate are becoming smaller and smaller, and may even become smaller than 6 mm in diameter. This means that both the spot size and the pitch between the spots decrease. The amount of fluid to be deposited decreases, while at the same time the accuracy of the positioning of the spots has to be much higher, preferably. Solution processing of the bioactive materials by ink jet printing is the preferred method of depositing the materials on the substrates. In this method, before drying, the solution penetrates into the membrane. Precision ink jet printing positions droplets within say 25-40 microns (mainly straightness errors). With a single nozzle print system it is possible to correct for straightness errors. For a multi-nozzle print head (which is particularly cost efficient) straightness errors may be intrinsic and random, and other means and methods have to be devised to compensate for these errors. Moreover, in order to ensure a good quality and efficiency of the high-throughput screening, it is desirable to bind as many labelled molecules of the analyte fluid as possible to those capture probes of the substrate that have binding capacity for the molecule considered. When binding of the molecules is insufficient (or hybridization in case of DNA strands), certain indicative factors may be missed and/or the fluorescent pattern may not be clearly distinguishable and/or may be deficient in some other sense. Although the known biological assay substrate as well as the method for producing such biological assay substrate yields a satisfactory binding efficiency, there is a need for a biological assay substrate as well as for a method for producing such biological assay substrate with improved binding efficiency. Moreover fast screening with increased speed of the analysis is desirable, especially in the field of clinical diagnostics.

### SUMMARY OF THE INVENTION

It is an object of the present invention to provide a porous biological assay substrate, including substrates for PCR and/or electrophoresis, with improved binding efficiency. It is a further object of the present invention to provide a method for producing such substrate by depositing a plurality of substances onto said substrate. It is a further object of the present invention to provide a device for producing such substrate which is able to deposit a plurality of substances onto said substrate. Another object of the present invention is to provide a method for examining analyte fluids, such as human blood or tissue samples, for the presence of certain bacteria, viruses and/or fungi, the method having an improved analysis efficiency.

The above objectives are accomplished by a porous biological assay substrate comprising a top layer provided with a plurality of holes in a pattern that matches the spot areas. Broadly speaking, by providing such a substrate, an increased binding efficiency of the substrate is obtained in comparison with the known biological assay substrates. Another advantage of the invention is that the analysis may be carried out faster, both for a flow-through and a flow-over configuration. According to the invention, the substrate is designed such that droplets emitted from a print head and landing next to the desired position are forced to the right position, a.o. by surface tension. The substrate of the invention is built-up of at least two layers, comprising a continuous top layer with holes in it according to a pattern and at the pitch of the desired spot pattern, and another layer being a porous membrane.

According to the invention, a method for producing a biological assay substrate is also provided. In the method for producing the biological assay substrate, a plurality of capture probe molecule solutions are released from at least one print head onto the porous substrate in plural spot areas, the method further comprising the step of providing the substrate with a top layer having a plurality of holes in a pattern that matches the spot areas, and compressing the assembly such that the substrate material between the holes is compressed and the substrate material in the spot areas partly fills the holes, whereby the top layer is at least partly bonded to the substrate. The layers of the composite substrate are preferably adhered to each other, more preferably by means of ultrasonic welding, allowing for a compaction of the membrane between the holes in the top layer while leaving the membrane material in the hole unaffected. Therefore a better definition of the material penetrating into the membrane is reached. The mechanical stiffness of the composite substrate of top layer and membrane is higher than the stiffness of the known substrate. This allows to use higher pressures when forcing the liquid containing the DNA strands through the substrate.

According to a preferred embodiment of the invention, the surface tension of the top layer can be regulated by chemical means, preferably by providing an anti-wetting coating, and/or by physical means, preferably by providing a surface structure with a micro-profile.

An additional advantage of the method and assay substrate according to the invention is that it requires less capture probes to be printed and/or needs less analyte fluid for a similar throughput than known hitherto. Both advantages reduce the cost of an analysis. Also, more fluid mixing and hybridization steps may be performed in order to improve the detection limit, thereby increasing analysis time. However according to a preferred embodiment of the invention the analysis time is decreased significantly compared to methods known in the art.

The present invention also provides a method for examining analyte fluids, such as human blood or tissue samples, for the presence of certain bacteria, viruses and/or fungi. In the method the analyte fluid is forced through or flows over a substrate according to the present invention. Flow-through is possible since the substrate material is porous. The binding of the target biological compound is the result of the free and/or forced flow of the sample fluid through and/or along the surface of the biological assay substrate, i.e. either from the lower surface to the upper surface or vice versa, and/or by a lateral flow from position A to position B on the substrate. To increase the chances for binding, flow-through may be repeated several times. The substrate of the present invention has the additional advantage that the number of such pumping cycles may be less for a similar binding efficiency.

As used herein, and unless stated otherwise, the term « microarray assay » designates an assay wherein a sample fluid, preferably a biological fluid sample (optionally containing minor amounts of solid or colloid particles suspended therein), suspected to contain target biological compounds is contacting (i.e. flowing over or flowing through) a biosensor solid substrate containing a multiplicity of discrete and isolated regions across a surface thereof, each of said regions having one or more probes applied thereto and each of said probes being chosen for its ability to bind specifically with a target biological compound. Notably, not every material deposited on the substrate needs to be a probe, i.e. has the ability to bind a specific analyte. The assay may also comprise other materials, such as materials used for calibration purposes, gridding markers, and so on. Such materials may already comprise a label.

As used herein, and unless stated otherwise, the term « analyte » or « target biological compound» designates a biological molecular compound fixed as a goal or point of analysis. It includes biological molecular compounds such as, but not limited to, nucleic acids and related compounds (e.g. DNAs, RNAs, oligonucleotides or analogs thereof, PCR products, genomic DNA, bacterial artificial chromosomes, plasmids and the like), proteins and related compounds (e.g. polypeptides, peptides, monoclonal or polyclonal antibodies, soluble or bound receptors, transcription factors, and the like), antigens, ligands, haptens, carbohydrates and related compounds (e.g. polysaccharides, oligosaccharides and the like), cellular fragments such as membrane fragments, cellular organelles, intact cells, bacteria, viruses, protozoa, and the like.

As used herein, and unless stated otherwise, the term « capture probe » designates a biological agent being capable to bind specifically with a « target biological compound» or « analyte » when put in the presence of or reacted with said target biological compound, and used in order to detect the presence of said target biological compound. Probes include biological molecular compounds such as, but not limited to, nucleic acids and related compounds (e.g. DNAs, RNAs, oligonucleotides or analogs thereof, PCR products, genomic DNA, bacterial artificial chromosomes, plasmids and the like), proteins and related compounds (e.g. polypeptides, monoclonal antibodies, receptors, transcription factors, and the like), antigens, ligands, haptens, carbohydrates and related compounds (e.g. polysaccharides, oligosaccharides and the like), cellular organelles, intact cells, and the like. Probes may also include specific materials such as certain biopolymers to which target compounds bind.

As used herein, and unless stated otherwise, the term « label » designates a biological or chemical agent having at least one physical property (such as, but not limited to, radioactivity, optical property, magnetic property) detectable by suitable means so as to enable the determination of its spatial position and/or the intensity of the detectable physical property such as, but not limited to, luminescent molecules (e.g. fluorescent agents, phosphorescent agents, chemiluminescent agents, electroluminescent agents, bioluminescent agents and the like), colored molecules, molecules producing colors upon reaction, enzymes, magnetic beads, radioisotopes, specifically bindable ligands, microbubbles detectable by sonic resonance and the like.

These and other aspects of the present invention will be apparent from and elucidated with reference to the embodiment(s) described hereinafter, taken in conjunction with the accompanying drawings, which illustrate, by way of example, the principles of the invention.

### BRIEF DESCRIPTION OF THE DRAWINGS

In the figures:
- Figure 1: illustrates schematically a biological test array obtainable by printing capture probes onto as substrate according to the present invention;
- Figure 2: illustrates schematically a biological assay device provided with a porous substrate according to the present invention;
- Figure 3A: represents schematically a precursor assembly for a first embodiment of a porous substrate according to the present invention;
- Figure 3B: represents schematically a first embodiment of a porous substrate according to the present invention;
- Figure 4A: represents schematically a precursor assembly for a second embodiment of a porous substrate according to the present invention; and
- Figure 4B: represents schematically a second embodiment of a porous substrate according to the present invention; and
- Figure 5: illustrates schematically a droplet misalignment after printing the first embodiment of the porous substrate according to the invention.

### DETAILED DESCRIPTION OF EMBODIMENTS

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn to scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

A preferred porous biological assay substrate according to the invention is characterized in that the density of the substrate material between the holes is higher than the density of the substrate material in the spot areas. This embodiment helps to prevent cross flow of the capture molecule solutions from one hole to an adjacent hole, thereby increasing the efficiency of the method for examining analyte fluids.

In another preferred embodiment of the porous biological assay substrate of the invention, at least part of the holes are tapered. Still more preferred the surface of the top layer is provided with anti-wetting properties. In one preferred embodiment, the anti-wetting properties are provided by an anti-wetting coating, which coating comprises even more preferably a fluoropolymer. In another preferred embodiment, the anti-wetting properties are provided by a micro-profiled surface structure.

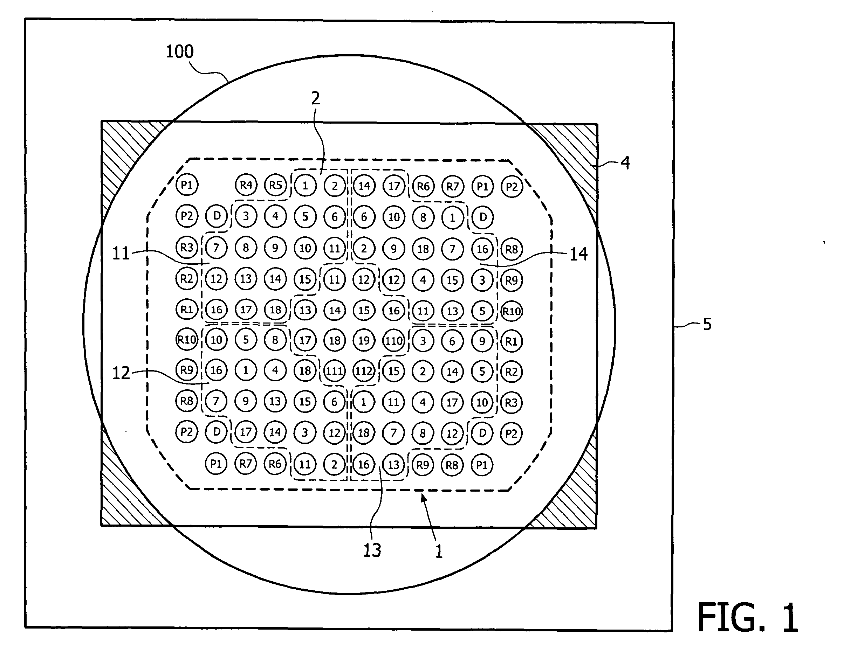

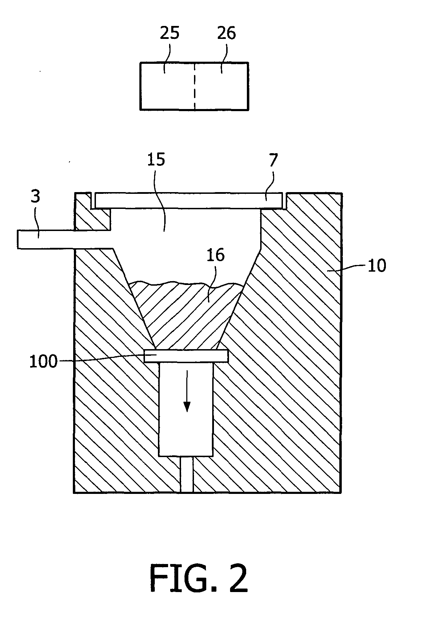

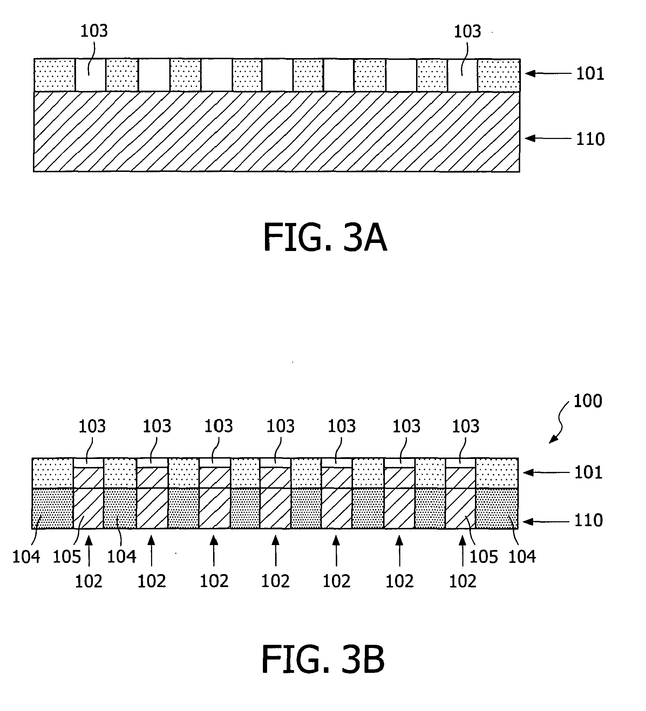

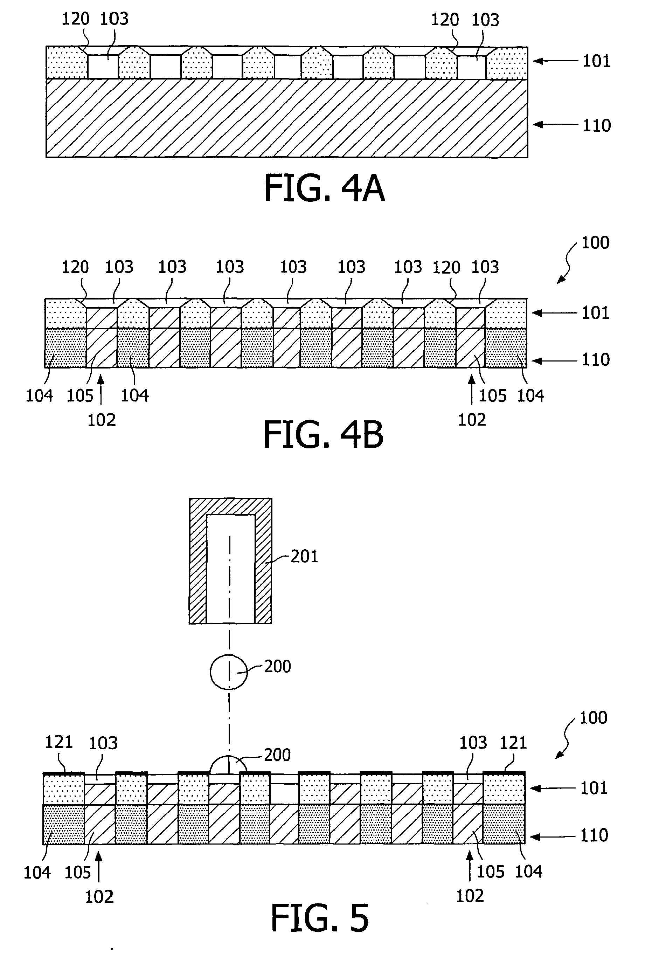

Figure 1 shows a biological test array (1) obtained by depositing, preferably by ink jet printing, a plurality of capture probe spots (2) on a porous substrate (100), such as a membrane. According to the invention, substrate (100) comprises a top layer provided with a plurality of holes in a pattern that matches the spot areas (as shown in figures 3B and 4B). In the example shown, the test array (1) is covered with a pattern of 128 spots (2) comprising 43 different bio-fluids, printed in a predefined pattern. The spots (2) are numbered, and each number represents a unique gene sequence or contains reference material. Note that the gene sequences occur in multiple duplicates in the array (1) on multiple mutually distant locations. The porous substrate (100) is fitted onto a supporting structure (4). Porous substrate (100) with the supporting structure or holder (4) is placed in a cartridge (5). A typical set-up is shown in figure 2. In a housing (10), a sample fluid (16) to be analysed is provided in a chamber (15) and pressure is applied at the inlet (3). This pressure forces the sample fluid (16) downwards through the porous solid substrate (100). A glass plate (7) permits an optical analysis of the solid substrate (100), if desired. Analyzing means (25) are provided for analyzing the solid substrate (100) for the presence of one or more target biological compounds. Said analysing means (25) may comprise a light source and an optical detection path, a lens, a filter, a digital camera etc in order to measure the optical fluorescence pattern. Other means (26) may be present, for instance for analyzing the solid substrates temperature, filled pore volume, and other desirably monitored parameters. The dashed line indicates that means (25) and (26) may eventually be combined into a single apparatus (e.g. an optical detection means such as a CCD camera or any other kind of optical detection device of which a camera is only one possibility. Other possibilities include photodetectors or a microscope. Provisions may be made to cycle the sample fluid (16) a number of times through chamber (15) and substrate (100). Preferably, the substrate (100) is continuously or intermittently but regularly wetted with the sample fluid (16). The analyte fluid is analysed for the presence of certain bacteria, viruses and/or fungi, by forcing it through the porous substrate. The free and/or forced flow of the sample fluid through the surface of the biological assay substrate, i.e. either from the lower surface to the upper surface or vice versa brings the analyte fluid in contact with the capture probes, which allows binding of the target biological compound to these capture probes able to bind with it. More in particular, the sample fluid (such as a PCR product) containing the different gene sequences characteristic for the DNA of different bacteria is brought into contact with the porous substrate (100) comprising the array of spots (2). Different DNA types (gene sequences) adhere to the different printed capture probes. In the embodiment shown in figure 1, different spots are visualised. The numbers 1 to 18 represent 9 different pathogens and 9 resistances. For reliability of the measurement, the same bio selective capture material may be printed in four different quadrants (11, 12, 13, 14) of membrane (100). In each of the quadrants (11, 12, 13, 14), spots of the same number have different neighbouring spots, preventing that less intense spots (2) are not detected because of overexposure from adjacent spots (2). Intensity calibration spots (R1 to R10) may be printed on the membrane (100), as well as four spots (D) in the corners of the membrane for intensity calibration distribution over membrane (100). PCR control spots (P1, P2) are also printed to validate the proper DNA-amplification by means of PCR. Along the sides of the membrane, preferably there is space available for printing identification dot patterns for tracing the micro array all the way from printing, packaging, storing (shelf life) and use.

A biological test array according to the invention preferably comprises a total amount of about 130 spots, as shown in figure 1, but may comprise many more spots, for instance more than 1000. Typical diameters of the spots are below 100 - 300 µm, but may be even lower, and they are positioned in a pattern with a pitch of typically less than 400 µm, preferably less than 300µm. Said spots are preferably printed in a periodic pattern e.g. in squared, rectangular or hexagonal configuration. Also a large amount of different bio-fluids (preferably 100 or more) are typically printed onto membrane (100).

As shown in figure 3B the porous substrate 100 is provided with capture probes 2 in plural spot areas 102. Substrate 100 further comprises a top layer 101 provided with a plurality of holes 103 in a pattern that matches the spot areas 102. Top layer 101 is positioned onto a membrane 110. A suitable membrane material is based on polyamide (nylon) although any suitable porous material may be used. The thickness of the membrane 110 may vary within a broad range. A typical thickness is about 150 mm. In a preferred embodiment the continuous top layer 101 has a thickness of about 50 mm, and is also made of a nylon. The holes 103 in the top layer may be made by any suitable method known in the art, laser drilling being the preferred method.

The biological assay substrate 100 is produced by a method, wherein the substrate 100 is provided with a top layer 101 having a plurality of holes 103 in a pattern that matches the spot areas 102. This assembly, shown in figure 3A is then compressed, such that the substrate material 104 between the holes 103 is compressed and the substrate material 105 in the spot areas 102 partly fills the holes 103, as shown in figure 3B. In the method, the top layer 101 is at least partly bonded to the substrate by providing ultrasonic energy. The method according to the invention results in a biological array substrate wherein the density of the substrate material 104 between the holes 193 is higher than the density of the substrate material 105 in the spot areas 102.

Figure 4B shows a second embodiment of the porous biological assay substrate, wherein at least part of the holes 103 have tapered sidewalls (120). Similar to what was discussed in relation to figures 3A and 3B, figure 4B shows a precursor substrate before the compressing and bonding step. In this embodiment the neighbourhood of the holes 103 is shaped such that a slightly misplaced droplet is guided towards the membrane 110. To make tapered holes, the same laser drilling process as used for making the cylindrical holes of figures 3A and 3B may be used.

As shown in figure 5 the porous biological assay substrate according to the invention is preferably characterized in that the surface of the top layer 101 is provided with anti-wetting properties. This results after drilling the holes 103 in a top layer of which at least the parts 121 between the holes 103 are provided with anti-wetting properties, preferably an anti-wetting coating of a fluoropolymer, and or a micro-profiled surface structure. The anti-wetting coating is preferably a thin (mono)-layer 121 of Teflon®-like material. This anti-wetting coating layer 121 could be coated or may be obtained through a mask plasma treatment, resulting in CF₄ groups on the surface, causing hydrophobic surface properties. This makes the upper surface of the composite structure of top layer 101 and membrane 110 anti-wetting. In another embodiment a micro-profiled surface structure (for instance a lotus leaf structure) is pressed into the top layer 101 before attaching the top layer 101 to the membrane 110. In still another embodiment arrays of nanotubes are applied onto the top layer 101, to obtain an anti-wetting surface. In this way a much better definition of the shape of the holes 103, provided with membrane material 105, is obtained, while at the same time the surface of the composite structure (101,110) has anti-wetting properties. At least partly due to these anti-wetting properties, droplets 200, emitted from a print head 201 and landed slightly next to the desired position (as shown in figure 5) are forced to flow to the hole 103.

Providing the surface of the top layer and/or the membrane with anti-wetting properties may be accomplished in a number of ways. In the embodiments described below, hydrophobic and/or hydrophilic regions are applied on top of the porous membranes in some pattern. The embodiments described have in common that they are based on deposition of at least a hydrophobic agent attached to the surface. Some of the embodiments make use of a mask and patterned deposition of said hydrophobic agent. Suitable methods are for instance described in US 5.904.824 "Microfluidic electrophoresis device" by BECKMAN INSTRUMENTS INC. Another embodiment uses patterned modification of said hydrophobic layer.

In an embodiment adapted to and suitable for oxidized surfaces, silanes are preferably used. It should be noted that other binding chemistry could be used as well however. In such embodiment, the surface of a porous polymeric membrane is first coated with Si or with a metal, and subsequently oxidized. This results in oxidized material at the surface, such as a surface containing SiOₓ or metal oxide groups. Silanes generally adhere very well to such surfaces. Siliceous surfaces, such as glass for instance, are naturally hydrophilic due to the presence of multiple hydroxyl groups which are attached to the tetravalent silicone atoms of the glass. According to this invention, these hydroxyl groups can undergo a dehydration reaction with an alkyl-chlorosilane or alkyl-alkoxysilane in a suitable anhydrous organic solvent to render the treated area hydrophobic. The silanol moiety of the glass forms a covalent bond with an organic monohalo silane such as a trialkyl-chloro silane or an alkyl-trialkoxy silane compound via a Si-O-Si bond. Thus, the surface properties of a glass substrate are modified by the alkylsilane group. Various silane derivatives, which are commercially available, are suitable for the practice of this invention.

Preferably, the alkyl or alkoxy groups on the silanes contain from 1 to about 30 carbon atoms. For example, octadecyl-dimethylchlorosilane is useful to produce the hydrophobic areas on the siliceous surfaces according to this invention. The silanizing agent can be dissolved in toluene or in another solvent. The solution can be applied to the siliceous areas to be rendered hydrophobic by roller or printing head and heated to evaporate the solvent. Any excess silanizing agent can be removed by washing with fresh organic solvents.

A surface having such "hydrophobic" (water repelling) regions in a predetermined pattern can be obtained by the use of a silane resistant mask. The areas covered by the mask are shielded from the silane. Only the areas exposed to the silane are rendered hydrophobic. This results in an anisotropic surface on a single substrate. In other words, a single substrate contains hydrophobic and hydrophilic regions in a pre-determined pattern. When an aqueous solution (such as the print solution comprising the capture probes) is subsequently applied on top of such an anisotropic surface, the aqueous solution will "wet" (adhere to) the surface in the pre-determined pattern. In a particular pattern, such hydrophobic surface can be formed all over the membrane surface except a matrix of spots which defines the micro array.

One alternative method of patterning a hydrophobic surface on any hydrated oxide surface involves applying a chemical by dip coating or spin coating. In this case, the chemical used renders the entire surface hydrophobic. The coated substrate can be subsequently baked, if necessary. Ultraviolet light exposure through a photo-mask or any other photo-resist technique, of certain desired area or pattern, will render the area exposed to ultraviolet "hydrophilic".

A suitable photo-sensitive chemical is based on 3-amino-propyl-trimethoxy-silane. A preferred photo-sensitive chemical is obtained by reacting the NHS-ester of polyethyleneoxide half acid with 3-amino-propyl-trimethoxy silane. The other carboxylic acid group is then reacted (after converting to primary amino terminus by treating with ethylene-diamine) with octadecyloxy-o-nitrobenzoic acid to obtain the photo silane compound. This compound will decompose to form hydrophilic area on the siliceous substrate upon exposure to 360 nm UV light. The o-nitrobenzoic acid decomposes via a well known mechanism. By washing the excess materials from the surface with appropriate "organic solvent", an "anisotropic" surface is obtained.

Thus, when a siliceous surface is coated with the photo silane compound, the entire surface is rendered hydrophobic. Then, a mask made of any material which does not transmit ultraviolet is applied, covering only the areas desired to remain hydrophobic. The coated siliceous surface is exposed to ultraviolet and the photo-silane decomposes to yield a hydrophilic surface in the exposed areas.

In another preferred embodiment photo cleavable SAM are photo-patterned. In this embodiment a photo mask is applied on top of a SAM-coated surface, and the surface UV irradiated, to fabricate hydrophilic and hydrophobic surface patterns on a membrane. A suitable UV light source is an Olympus Epi-Fluorescent Microscope (BX-60) passed through a near-UV filter cube (U-MNUA, type BP 360-370 nm) with a band pass of 360- to 370-nm wavelength. After irradiation, for instance during 90 min., and rinsing and drying the surface different molecular structures are obtained in the irradiated and unirradiated regions. The photo cleavable materials may be copolymerized when making the porous membranes.

It is also possible to irradiate the surface with ultraviolet light comprising reactive gasses, such as O₂, H₂O or HF₄ for instance. Another option is to locally take or burn away surface material by irradiating with a laser bundle.

Suitable porous substrates may include a network having a plurality of pores, openings and/or channels of various geometry and dimensions. Porous substrates may be isotropic or anisotropic, unidirectional or multidirectional. Porous substrates may be nanoporous or microporous, i.e. the average size of the pores, openings and/or channels may suitably be comprised between about 0.05 µm and about 10.0 µm. In one embodiment this average pore size may be between 0.1 µm and 3.0 µm. In another embodiment, the average pore size may be between about 0.2 and 1 µm. The term "porosity" usually means or includes the ratio of the volume of all the pores or voids in a material with respect to the volume of the whole material. In other words, porosity is usually the proportion of the non-solid volume to the total volume of material. In the sense of the present invention, the term "open porosity" (also called effective porosity) especially means or includes the fraction of the total volume in which fluid flow is effectively taking place. Since the open porosity alone is of importance in the context of the present application (closed pores are not accessible to capture probe and sample fluids) the terms "porosity" and "open porosity" are used interchangeably in the present application, unless explicitly noted otherwise. In the sense of the present invention porosity is especially a fraction between 0 vol.% and 100 vol.%. According to a preferred embodiment said porosity is ranging from 20 vol.% to 98 vol.%, more preferably from 30 vol.% to 80 vol.% and most preferably from 40 vol.% to 70 vol.%.

According to a preferred embodiment of the present invention, the porous substrate material is chosen from the group comprising
- amorphous polymers, preferably from the group comprising PC (polycarbonate), PS (polystyrene), PMMA (polymethylmethacrylate), polyacrylates, polyethers, cellulose ester, cellulose nitrate, cellulose acetate, cellulose or mixtures thereof,
- semicrystalline polymers, preferably from the group comprising PA (polyamide= nylon materials), PTFE (polytetrafluoroethylene), polytrifluoroethylene, PE (polyethylene), PP (polypropylene), or mixtures thereof,
- rubbers, preferably from the group comprising PU (polyurethane), polyacrylates, silane based polymers such as PDMS (polydimethylsiloxane) or mixtures thereof,
- gels (= polymer networks with fluid solvent matrix), preferably from the group comprising agarose gel, polyacrylamide gel or mixtures thereof
- metals (such as aluminum, tantalum, titanium), alloys of two or more metals, doped metals or alloys, metal oxides, metal alloy oxides or mixtures thereof
or mixtures thereof. These materials have shown to be suitable materials within the present invention.

The thickness of the substrate is not a limiting feature of this invention and it can vary from about 50 nm up to about 3 µm or higher, e.g. up to 1 mm. If the membrane is freestanding, e.g. in the case of a flow-through device (as described above) the substrate thickness can range from 1 µm to hundreds of µm, e.g. from 20 µm to 400 µm, or from 50 µm to 200 µm.

The shape and or size of the substrate, e.g. the membrane, are not considered to be limiting features of the present invention. It may be circular, e.g. with a diameter ranging between about 3 and 15 mm, but any other substrate shape (rectangular, square, oval,...) and/or size may also be suitable.

The probes used for the present invention should be suitably chosen for their affinity to the target biological compounds or to the relevant modifications of said target biological compounds suspected to be present in the sample to be analyzed. For example, if the target biological compounds are DNA, the probes can be, but are not limited to, synthetic oligonucleotides, analogues thereof, or specific antibodies. A non-limiting example of a suitable modification of a target biological compound is a biotin substituted target biological compound, in which case the probe may bear an avidin functionality.

In a particular embodiment of the present invention, several different probes are deposited into and/or onto the substrate. In a more specific embodiment, multiple different probes are spotted in an array fashion on physically distinct locations along one surface of said solid substrate in order to allow measurement of different target biological compounds in parallel. This embodiment is usually named a micro-array. In order to more easily support subsequent detection and identification, one or more additional spots (e.g. for intensity calibration and/or position detection) can be spotted as well onto the surface of the substrate material. Spotting can be suitably effected by any methods known in the art such as, but not limited to, ink-jet printing, piezoelectric spotting, robotic contact printing, micropipetting, and the like. Following spotting, the probes become immobilized onto the surface of the substrate material, either spontaneously due to the substrate (e.g. membrane) inherent or acquired (e.g. via activation) properties, or through an additional physical treatment step (such as, but not limited to, cross-linking, e.g. through drying, heating, a temperature treatment step, or through exposure to a light source).

According to the invention, a method for producing a biological assay substrate, wherein a plurality of capture molecule solutions are released from at least one print head onto the porous substrate is provided, the method comprising the step of providing the substrate with a top layer having a plurality of holes in a pattern that matches the spot areas, and compressing the assembly such that the substrate material between the holes is compressed and the substrate material in the spot areas partly fills the holes, whereby the top layer is at least partly bonded to the substrate.

In the method according to the invention any substrate having any degree of porosity may in principle be used. Preferred substrates include porous substrates with a broad pore size distribution. Even more preferred substrates include those having porosity morphologies comprising interconnected and/or multidirectional pores. Such preferred substrates generally exhibit differences in flow of a certain medium there through, depending on whether the substrate and the medium are dry-wet, wet-wet, or wet-dry respectively. A preferred species of a substrate comprises a membrane of a suitable polymer. Multi- and unidirectional porous membranes are known in the art, but not in connection with the method according to the invention, and are commercially available. Moreover charged and supercharged, and/or chemically functionalized membranes are preferably used according to the invention.

The invention also relates to an ink jet device for producing such biological assay substrate and to a biological assay substrate obtainable by the method. The substance, comprising biologically active molecules, is preferably dissolved in a solution. This solution is typically a fluid, like water or different types of alcohol, and may also contain small amounts of additives, for instance to adjust the surface tension, viscosity or boiling point, in order to optimise print characteristics, spot formation, shelf life of the bio-fluids, and so on.

While the present invention has been illustrated and described with respect to particular embodiments and with reference to certain drawings and foregoing description, such illustration and description are to be considered illustrative or exemplary and not restrictive. The invention is not limited to the described embodiments. Instead, the ink jet printer according to the present invention can be used for any precision placement of droplets onto membranes. It is particularly suited for the production of biosensors for molecular diagnostics. Diagnostics include rapid and sensitive detection of proteins and nucleic acids in complex biological mixtures, such as blood or saliva, for on-site testing and for diagnostics in centralized laboratories. Other applications are in medical (DNA/protein diagnostics for cardiology, infectious disease and oncology), food, and environmental diagnostics.

## Claims

1. A porous biological assay substrate, suitable for detecting at least one analyte in at least one sample fluid, the substrate being provided with capture probes in plural spot areas, the capture probes being able to each specifically bind at least one target analyte, wherein the substrate comprises a top layer provided with a plurality of holes in a pattern that matches the spot areas.

2. A porous biological assay substrate according to claim 1, wherein the density of the substrate material between the holes is higher than the density of the substrate material in the spot areas.

3. A porous biological assay substrate according to claim 1 or 2, wherein at least part of the holes are tapered.

4. A porous biological assay substrate according to any one of the preceding claims, wherein the surface of the top layer is provided with anti-wetting properties.

5. A porous biological assay substrate according to claim 4, wherein the anti-wetting properties are provided by an anti-wetting coating.

6. A porous biological assay substrate according to claim 5, wherein the anti-wetting coating comprises a fluoropolymer.

7. A porous biological assay substrate according to claim 4, wherein the anti-wetting properties are provided by a micro-profiled surface structure.

8. Method for producing a biological assay substrate, wherein a plurality of capture probe molecule solutions are released from at least one print head onto the porous substrate in plural spot areas, the method comprising the step of providing the substrate with a top layer having a plurality of holes in a pattern that matches the spot areas, and compressing the assembly such that the substrate material between the holes is compressed and the substrate material in the spot areas partly fills the holes, whereby the top layer is at least partly bonded to the substrate.

9. Method according to claim 8, wherein the top layer is bonded to the substrate by providing ultrasonic energy.

10. Method according to claim 8 or 9, wherein the capture molecule solutions comprise a biochemical reactant and/or an oligonucleotide, and/or a polypeptide and/or a protein, and/or a cell, and/or (parts of) RNA/PNA/LNA.

11. A porous biological assay substrate obtainable by the method of any one of claims 8 - 10, comprising one or more capture probes in plural spot areas, which capture probes are able to each specifically bind one target analyte, wherein the substrate comprises a top layer provided with a plurality of holes in a pattern that matches the spot areas.

12. Method for examining analyte fluids, such as human blood or tissue samples, for the presence of certain bacteria, viruses and/or fungi, wherein the analyte fluid is forced through or over a substrate according to any one of claims 1-7.

13. Method for examining analyte fluids, such as human blood or tissue samples, for the presence of certain bacteria, viruses and/or fungi, wherein the analyte fluid is forced through or over a substrate, obtained by a method according to any one of claims 8 - 10.
